(19) **Europäisches Patentamt / European Patent Office / Office européen des brevets**

(11) **EP 4 362 026 A1**

(12) # EUROPEAN PATENT APPLICATION

(43) Date of publication:
01.05.2024 Bulletin 2024/18

(21) Application number: 22203671.7

(22) Date of filing: 25.10.2022

(51) International Patent Classification (IPC):
G16B 15/30 (2019.01)   G16B 20/30 (2019.01)
G16B 20/20 (2019.01)

(52) Cooperative Patent Classification (CPC):
G16B 15/30; G16B 20/20; G16B 20/30

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(71) Applicant: Max-Planck-Gesellschaft zur
Förderung der
Wissenschaften e.V.
80539 München (DE)

(72) Inventors:
• **Hummer, Prof. Dr. Gerhard**
**60438 Frankfurt am Main (DE)**

• **Sikora, Dr. Mateusz**
**60438 Frankfurt am Main (DE)**
• **Bülow, Sören von**
**60438 Frankfurt am Main (DE)**
• **Blanc, Dr. Florian**
**60438 Frankfurt am Main (DE)**
• **Beck, Dr. Martin**
**60438 Frankfurt am Main (DE)**
• **Covino, Dr. Roberto**
**60320 Frankfurt am Main (DE)**

(74) Representative: **Dentons Patent Solutions
Rechtsanwaltsgesellschaft mbH
Jungfernturmstraße 2
80333 München (DE)**

(54) **PREDICTION OF MUTATION HOT SPOTS IN ANTIGENS**

(57) The present invention relates to a computer-implemented method of predicting site-specific mutation activity of an antigen, said method comprising (a) providing in silico at least one three-dimensional structure of said antigen, preferably an ensemble of three-dimensional structures of said antigen; (b) providing in silico at least one three-dimensional structure of at least one antibody or fragment thereof capable of binding to or specific for said antigen, preferably an ensemble of three-dimensional structures of said antibody or fragment thereof; (c) determining in silico the accessibility of the structure(s) of (a) to the structure(s) of (b), wherein higher accessibility is indicative of higher mutation activity, in particular under selective pressure exerted by said antibody or fragment thereof; thereby predicting said site-specific mutation activity.

EP 4 362 026 A1

Figure 1

## Description

The present invention relates to a computer-implemented method of predicting site-specific mutation activity of an antigen, said method comprising (a) providing in silico at least one three-dimensional structure of said antigen, preferably an ensemble of three-dimensional structures of said antigen; (b) providing in silico at least one three-dimensional structure of at least one antibody or fragment thereof capable of binding to or specific for said antigen, preferably an ensemble of three-dimensional structures of said antibody or fragment thereof; (c) determining in silico the accessibility of the structure(s) of (a) to the structure(s) of (b), wherein higher accessibility is indicative of higher mutation activity, in particular under selective pressure exerted by said antibody or fragment thereof; thereby predicting said site-specific mutation activity.

In this specification, a number of documents including patent applications and manufacturer's manuals are cited. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

The immune system is generally capable of recognizing pathogens as well as new variants of pathogens and of mounting a response thereto. Biotechnological means to stimulate the immune system such as vaccines as well as therapeutics based on components of the immune system such as therapeutic antibodies are constantly being developed and improved. Yet, insufficient responses of the immune system and insufficient stimulatory effect of a vaccine have been and remain challenges which are difficult to meet. This is familiar, for example, from recurring Influenza infections and has become evident with the Covid-19 pandemic.

Pathogens such as viruses and bacteria constantly probe the phenotypic effect of variations in their genetic information. This makes it difficult to develop vaccines for infections yet to come or variants yet to appear. Moreover, both the immune system as well as vaccines and therapeutic antibodies exert a selection pressure in response to which in particular those variants of pathogens prevail that escape the immune system, a phenomenon also called immune evasion.

The COVID-19 pandemic caused by the severe acute respiratory syndrome coronavirus-2 (SARS-CoV-2) has entered a new phase with the advent of the Omicron variants (BA.1 to BA.5). Of the several past and present variants of concern (VoC), Omicron BA.1 was the first escape variant, with significant reduction of immunity conferred by previous infection with earlier variants or by vaccination. The induction of cross-neutralizing antibodies against Omicron infection from sera collected from patients previously infected with earlier VoC was strongly reduced. Reduced vaccine effectiveness is alleviated by additional vaccination ("booster") doses.

Spike (S) protein, a homo-trimeric fusion glycoprotein exposed on the surface of the virus is the main target of the natural or stimulated immune response and thus is subjected to the greatest immune pressure. Omicron BA.1 has 39 changes in its spike protein counting each mutated, deleted, or inserted amino acid. Many of these changes are located in the receptor binding domain (RBD), which in SARS-CoV-2 spike is found in either closed (i.e., largely protected from the immune system) or open conformation, in which it can bind to the human angiotensin-converting enzyme 2 (ACE2) receptor and thus mediate attachment to and entry into the cell. Omicron has since further evolved through BA.2 into BA.4/BA.5, with 11 additional mutations acquired and 14 mutations lost with respect to BA.1.

An understanding of the factors driving the mutations in the spike protein and the ability to predict the sites of new mutations in future variants is of tremendous interest, given the impact of emerging variants on the trajectory of the pandemic. Deep mutational scanning results indicate that the sublineages BA.2.12.1, BA.4 and BA.5 show neutralization evasion against both vaccination (with wild type spike) and from BA.1 infection (Cao et al., Nature 602(7898): 657-663, 2022). This suggests that Omicron keeps evolving to evade the response to the previously prevalent variant. A recent study used a deep mutational learning technique to assess the impact of RBD mutations on RBD-ACE2 binding from a yeast screening library (Taft et al., Cell doi:10.1016/j.cell.2022.08.024, 2022). The authors predicted a range of future antibody-escape RBD variants. Previously, Bai et al. predicted the effect of spike mutations from calculated binding free energies of complexes of spike with antibodies and the ACE2 receptor based on a coarse-grained model (Bai et al., Journal of the American Chemical Society 143(42): 17646-17654, 2021). Chen et al. used short molecular dynamics (MD) simulations of spike variants to train a classification network in order to predict binding free energy changes of the RBD-ACE2 interactions (Chen et al., Proceedings of the National Academy of Sciences USA 118(42): e2106480118, 2021). Starr et al. used deep mutational scanning to systematically address RBD mutation effects on protein expression, ACE2 interaction, and antibody recognition (Starr et al., Cell 182(5): 1295-1310.e20, 2020; Starr et al., Science 371(6531): 850-854, 2021). Recently, a structural investigation found that RBD-ACE2 binding affinity of the Omicron variant is similar to the Delta variant, and that Omicron spike displays significant antibody evasion (Mannar et al., Science 375(6582): 766-764, 2022).

In an earlier study co-authored by the present inventors (Sikora et al., PLoS Comput Biol 17(4): e1008790, 2021), a computational epitope map of SARS-CoV-2 spike protein has been provided. This study made use of a composite scoring scheme based on accessibility, rigidity, conservation, and immunogenicity scores. The study is silent about the prediction of mutation hotspots or immune evasion. Surprisingly, a conceptually simpler scoring scheme than that of Sikora et al., *loc. cit.,* has now been found by the present inventors to be superior to the earlier composite scoring scheme in terms of identification and prediction of mutation hot spots.

In view of the prior art and its shortcomings, the technical problem underlying the present invention can be seen as the provision of improved means and methods for predicting mutation hotspots in antigens, including antigens in variants of concern of pathogens with pandemic potential.

This technical problem has been solved by the subject-matter of the claims, as demonstrated by the Examples.

Accordingly, in a first aspect, the invention provides a computer-implemented method of predicting site-specific mutation activity of an antigen, said method comprising

(a) providing *in silico* at least one three-dimensional structure of said antigen, preferably an ensemble of three-dimensional structures of said antigen;
(b) providing *in silico* at least one three-dimensional structure of at least one antibody or fragment thereof capable of binding to or specific for said antigen, preferably an ensemble of three-dimensional structures of said antibody or fragment thereof;
(c) determining *in silico* the accessibility of the structure(s) of (a) to the structure(s) of (b), wherein higher accessibility is indicative of higher mutation activity, in particular under selective pressure exerted by said antibody or fragment thereof;

thereby predicting said site-specific mutation activity.

The term "antigen" has its art-established meaning. It refers to a molecule such as a polypeptide, protein, a post-translational modification such as a polysaccharide and any combination thereof that is capable of eliciting an immune response. Antigens are recognized and bound *inter alia* by antibodies or fragments thereof. Antigens, in particular those of pathogens, are known to evolve. Owing to a limited precision of the machinery for replicating the genetic material, there is a constant probing of new variants. Variants which gain an advantage under the circumstances given will generally prevail. Of interest here are those circumstances where the mammalian immune system exerts a selection pressure. Elements exerting such pressure include antibodies raised against a pathogen including those antibodies which are generated in response to a vaccination.

For the purpose of designing vaccines it is of interest to know which particular regions or epitopes of an antigen encoded by a pathogen are likely to undergo modifications, given that such modifications may lead to immune evasion, the consequence being that a previously effective vaccine fails to trigger a satisfactory response to a new variant of the pathogen. The present invention focuses on the identification or prediction of such regions where elevated mutational activity is to be expected, especially under the selection pressure of components of the mammalian immune system. In other words, the present invention allows for assessing the fitness of a given antigen in terms of its ability to escape antibody-based immune response.

Such fitness information is obtained at high structural resolution, generally at the amino acid level. Of note, and as shown in the Examples, the method of the present invention successfully predicted new variants of SARS-CoV-2. This has been achieved with only limited structural and genetic information of the virus available at the onset of the pandemic. Such information is extremely valuable when designing and developing counter-measures against a pathogen, especially against a pathogen capable of causing severe disorders and/or with pandemic potential. In a significant number of instances, such conter-measures are related to the immune system, such as vaccines, therapeutic vaccines and therapeutic antibodies.

To that end, *in silico* representations of biomolecular structures, in particular polypeptide and protein structures are employed. Such representations are datasets which can be stored on a computing device or a data carrier. For example, such representation can be a list of three-dimensional coordinates of the constituent atoms and/or groups of atoms of the molecule of interest. Coordinates may be Cartesian coordinates, but do not have to be. Any coordinate system defining a three-dimensional space such as polar coordinates may be used. Owing to decades of structure determination and prediction efforts, a host of three-dimensional structure representations are available or can be generated; for databases and methods see further below.

Within *in silico* structures, a distinction can be made between representations which provide a single representative set of coordinates which correspond to a single structure on the one hand and datasets on the other hand, where more than one set of coordinates is provided for a given molecule. The latter case is also referred to as an ensemble of structures. Of particular interest here are those ensembles of structures which are representative of the conformational space available to a given molecule. It is well established that proteins and polypeptides are flexible, the consequence that a single structure may be representative, but lacks information about flexibility. Even though the present method may be implemented with such single representative structures, preference is given to ensembles of structures.

As disclosed in more detail below, ensembles of structures can be generated from a single starting structure, or the information about flexibility contained in a starting ensemble of structures may be further enriched, thereby converting such starting ensemble of structures into a final ensemble of structures, the latter containing a greater number of structures and/or more complete information about the conformational space available to a given molecule. In either case, conformational sampling methods may be employed. A preferred method of conformational sampling is molecular dynamics simulation (MD). An example of a starting ensemble of structure is an ensemble of NMR structures, given that the results of structure determination by NMR a generally represented as ensembles.

The term "antibody" has its art-established meaning and generally refers to elements of the humoral immune response the immune system is capable of raising against an antigen, e.g. to combat an infection. In addition to antibodies as they are formed by the immune systems, antibody fragments may be employed, in particular those antibody fragments which contain antigen binding sites or complementarity determining regions (CDRs). Such fragments are well known and include Fab, $F(ab')_2$, scFv, dsFv, ds-scFv and diabodies.

Antibodies and fragments thereof are accordingly capable of binding to a given antigen. Preferably such binding is specific, i.e., the affinity of an antibody or fragment thereof for a given antigen (the cognate antigen) is higher than for any other possible antigen.

Step (c) of the method of the first aspect provides for determining the accessibility of the structure(s) of (a) and the structure(s) of (b) to each other, preferably of the constituent building blocks of the molecules under consideration to each other. In other words, what is determined is the accessibility of said antigen to said antibody or fragment thereof, also abbreviated herein as AA (antibody accessibility), and a score determined by the method of the invention is also referred to as antibody accessibility score (AAS).

In a preferred embodiment, the accessibility of the amino acid residues of said antigen to the amino acid residues of said antibody or fragment thereof is assessed. On the other hand, post-translational modifications (PTMs) may be taken into account where desired; for details see further below.

Generally speaking, accessibility is a measure of proximity. In other words, if, for a given antigen structure and a given antibody or fragment structure, a predefined proximity between an amino acid of said antigen and any amino acid of said antibody or fragment is possible in three-dimensional space, then said amino acid of said antigen is considered to be accessible for said antibody or fragment. As detailed further below, zooming in on specific parts of the antibody or the antigen is envisaged. For example, within the antibody or fragment thereof, the CDRs may be considered separately such that it can be determined whether a given amino acid of the antigen is accessible to said CDRs.

Accessibility may be assessed at various levels, depending on whether the structure representation for this purpose is fine-grained or coarse-grained. For example, accessibility (and the underlying proximity) may be assessed on an atomic level. On the other, it may be assessed at the level of larger building blocks, in the case of polypeptides and proteins preferably at the amino acid level. Further details in that respect are disclosed further below.

Accessibility may be influenced by several factors, the consideration of which is key to the invention. These factors include flexibility of the molecules (antigen and antibody or fragment thereof) and the presence of non-amino acid building blocks, in particular post-translational modifications.

The consequence of flexibility is that different conformations are available to a molecule. While in one conformation a given amino acid or atom of an antigen may be accessible to an antibody or fragment thereof, this might not be the case for a different conformation of the same antigen or the same antibody or fragment. While accessibility in accordance with the invention may be assessed on the basis of single structures, preference is given to the use of ensembles of structures. When using ensembles of structures for the purpose of determining accessibility, one way is to determine accessibility for each possible pairing of structures from either ensemble. For example, if an ensemble of antigen structures contains n members, and an ensemble of antibody or fragment structures contains $m$ members, $n \times m$ pairings are possible and each of these pairings the accessibility of each amino acid or atom of the antigen to each amino acid or atom of the antibody can be determined.

Without wishing to be bound by a specific theory, it is considered that ensembles represent the conformational space available to a given molecule such that determining accessibility using ensembles yields a more realistic picture of the accessibility in reality. Accessibility in turn, and again without wishing to be bound by a specific theory, is considered to correlate with the likelihood of a given region, site, epitope or amino acid of the antigen to change or mutate. This is because the more accessible an amino acid or epitope is to an antibody, the more likely a pathogen may benefit from a mutation of said amino acid or epitope such that recognition by said antibody is impaired.

Since mutations alter the amino acids of an antigen, and CDRs of an antibody are formed by amino acids, the method of the present invention preferably provides for determining of mutual accessibility at the amino acid residue level. Yet, proteins and polypeptides may, and in many instances do, comprise non-amino acid constituents. After synthesis on the ribosome, a polypeptide or protein is often subject to post-translational modifications (PTMs). Without wishing to be bound by a specific theory, it is considered that said PTMs provide less contribution to the antigenicity of an antigen than the amino acids of said antigen, and less contribution to the capability of an antibody or fragment to recognize an antigen. The mention of "less contribution" reflects a gradual phenomenon. Certain PTMs might contribute to antigenicity while other do less and yet others do not. In one approach (which turned out to be very successful; see the Examples), PTMs have not been considered at all when determining mutual accessibility of structures. Yet, the invention is not so limited.

In other words, in a preferred embodiment, the present invention may be performed with antigens and antibodies based on their amino acid composition only. This may apply in particular in those instances where PTMs do not occur or there is no knowledge about them. Preference is given, though, to a proper consideration of PTMs, be it on the antigen or the antibody or fragment. In a preferred embodiment, PTMs can be considered as additions to the antibody and/or to the antigen in their respective ensembles. Preferably, each member of the ensembles of antibody or fragment and/or antigen structures containing PTMs can be extended by considering multiple combinations and conformations of said PTMs.

In view of the considerations above, PTMs on either molecule may provide a shielding effect on those interactions (amino acid-amino acid interactions) which are likely to be relevant for immunity and immune evasion. That is, residues which are shielded, in a single structure or over time, are less likely to be mutational hot spots. "Shielded" is equivalent to not or only partially accessible, the concept of partial accessibility referring to those implementations of the invention which employ an ensemble of structures and/or when multiple PTMs are considered for each structure. A given residue may be accessible in a fraction of the structures comprised in an ensemble under consideration, while in another fraction not.

While it is preferred to use ensemble representations of the respective entire structures, it is also envisaged, preferably for the purpose of capturing the above-mentioned shielding effect, to use a single structure in terms of the amino acid chain(s), but sample the conformational space available to PTMs; see, for example doi.org/10.1101/2021.08.04.455134.

Surprisingly, the present invention which preferably uses only mutual accessibility of antigen and antibody or fragment, outperforms methods of the prior art which either use solvent accessibility or more involved composite scoring schemes. Indeed, it is unexpected that a measure reflecting purely the physical accessibility in steric terms (not to water, but to the cognate binding molecule) yields the performance which is evidenced by the Examples.

In other words, the concept of accessibility as defined herein alone provides for superior performance.

Yet, a further advantage of the invention becomes apparent when PTMs are present and factored in when determining accessibility. As mentioned above, the handling of PTMs may be adjusted, preferably based on previous knowledge about whether a given PTM is known or expected to contribute to antigenicity or whether its predominant role is rather the shielding of those structural elements, in particular amino acid residues, which contribute to antigenicity. As mentioned, in one preferred embodiment, PTM-PTM contacts and PTM-amino acid contacts are not considered when determining accessibility. As an alternative, PTMs may be considered, in particular in those cases where a contribution thereof to antigenicity is known or expected.

In a preferred embodiment, said antigen, said antibody and/or said fragment thereof comprise post-translational modifications, preferably selected from N-, O- and S-glycosylation, C-mannosylation, glycation, acylation, alkylation, butyrylation, malonylation, propionylation, sulfation, phosphorylation, ubiquitination, and sumoylation.

It is understood that said PTMs are comprised in said structures or ensembles of structures, more specifically in the *in silico* representations thereof in accordance with the invention.

The above non-exhaustive list of PTMs refers to common and well-known PTMs.

In a further preferred embodiment, said ensemble(s) is/are representative of the structural diversity of said antigen, said antibody and/or said fragment thereof at temperatures occurring in a mammalian, preferably human organism and/or at temperatures from 273 to 323 K such as from 300 to 310 K.

This embodiment provides for the simulation of conditions which are typical for those environments where interaction occur which are relevant to immunity, the raising of antibodies and immune evasion.

In a further preferred embodiment, said structure(s) or said ensemble(s) of (a) and/or (b) are obtained from a database of three-dimensional structures, by crystallography, by NMR spectroscopy, by electron microscopy, by mass spectrometry, by atomic force microscopy, and/or by *in silico* structure prediction.

This is a non-exhaustive list of common sources of three-dimensional structure information. Whatever is contained in the mentioned database may be obtained by any of the list methodologies. For example, an entry in a three-dimensional structure database may be obtained, for example, by X-ray crystallography or NMR spectroscopy, or, when practicing the invention, X-ray crystallography or NMR spectroscopy may be employed in a first step to obtain an *in silico* representation of a structure or an ensemble of structure, which subsequently is fed into the method of the first aspect as defined above.

*In silico* structure prediction methods are known in the art and include prediction from protein amino acid sequence and/or fragmentary or low resolution structural information using methods such as homology modeling, integrative modeling, or artificial intelligence-based methods such as AlphaFold; see, for example, Tunyasuvunakool et al., Nature 596: 590-596 (2021).

Databases of three-dimensional structures are known in the art and include the Protein Data Bank (PDB), see Berman et al., Nucleic Acids Research 28: 235-242 (2000), servers being operated by the Research Collaboratory for Structural Bioinformatics (RCSB).

In a further preferred embodiment, wherein conformational sampling of said structure(s) is performed and/or of said ensemble(s) is extended by computer simulation such as molecular dynamics simulation and/or Monte-Carlo simulation, preferably on or representative of a microsecond timescale. Overviews of conformational sampling methods can be found in Liwo and Scheraga, Current Opinion in Structural Biology 18: 134-139 (2008), and in Jorgensen and Tirado-Rives, J. Phys. Chem. 100(34): 14508-14513 (1996). A description of Monte-Carlo simulation can be found in Linke M, Quoika P, Bramas B, Kofinger J, Hummer G. Complexes: Efficient and versatile coarse-grained simulations of protein complexes and their dense solutions. ChemRxiv. Cambridge: Cambridge Open Engage; 2022. A preferred implementation of molecular dynamics simulation is described in Abraham et al., SoftwareX, 1-2: 19-25 (2015).

As stated above, ensembles as such are considered to provide information about the conformations and flexibility available to a given molecule. Yet, further conformational sampling, being it beginning with a single structure or starting already from an ensemble may be enhanced *in silico* by simulation methods. Particularly preferred in accordance with the invention is molecular dynamics simulation. In terms of timescales, preference is given to timescales to be covered by a simulation which correspond to the known or expected timescale of motion available to a given molecule. As a rule of thumb, the bigger a molecule is, the more likely it is that motions on a slow timescale are available to and performed by it. As a consequence, computational requirements dramatically increase with the size of the system to be simulated, first because of the size as such, and secondly in view of the slow motions performed by such systems. The Examples demonstrate MD simulation of an antigen antibody system where sampling has been performed on a microsecond timescale.

Preferred durations of the MD simulation are from 0.1 to 1000 $\mu$s, from 0.5 to 100 $\mu$s or from 1 to 10 $\mu$s.

The series of time frames obtained by MD will then be used as input for the method of the first aspect. The term "time frame" refers to the set of three-dimensional coordinates of a structure at a given time point during the MD simulation.

It is preferred to perform MD simulation for the antigen. As disclosed further below, for the antibody or fragment thereof a rigid structure or an ensemble of rigid structures may be used (said ensemble in turn being representative of flexibility of the antibody or fragment).

On the other hand, it is envisaged to perform conformational sampling, e.g. by MD simulation, of the antibody or fragment as well in order to enrich the information about the conformational space available to said antibody or fragment.

In a further preferred embodiment, said determining in step (c) is effected by *in silico* docking of said structure or members of said ensemble of (a) with said structure or members of said ensemble of (b).

"Docking" as used herein generally refers to a computational method which involves bringing two or more molecules (i.e., their *in silico* representations) into proximity or allow that said molecules approach each other; see, for example, Amaro, Biophysical Journal 114: 2271-2278 (2018); Fan et al., Quant. Biol. 7: 83-89 (2019); Pinzi and Rastelli, Int. J. Mol. Sci. 20: 4331 (2019); and Krawczyk et al., Bioinformatics 30: 2288-2294 (2014). As such, docking simulates a binding process such as the binding between an antigen and an antibody or fragment thereof. Whether and where such binding occurs depends on the features of said structures. Since binding implies close proximity, docking is a means of determining accessibility in accordance with the invention. If a region of an antigen cannot approach any region of an antibody or fragment, docking of said region to said antibody or fragment is not possible and accessibility of said region does not exist. A preferred docking method is described in Sikora et al., PLoS Comput Biol 17(4): e1008790 (2021), and in Kim and Hummer, J. Mol. Biol. 375: 1416-1433 (2008).

When performing docking, all possible orientations of the molecules involved as well as all direction of approach are preferably being sampled. It is also possible to focus on specific regions such as CDRs on the one hand and specific epitopes on the other hand.

In a further preferred embodiment, said docking is rigid-body docking.

Rigid-body docking provides for a rapid assessment of accessibility. While seemingly limiting the influence of the above discussed flexibility of the binding partners, this is actually not the case. Since docking, in particular if ensembles are used, employs multiple pairings, binding options and accessibilities which are not available to all conformations, but only a part thereof are sampled. Thereby, flexibility is accounted for.

It is understood that for a plurality of said multiple pairings, preferably for all pairings, docking is performed. For example, for an ensemble of *n* antigen structures and an ensemble of *m* antibody structures, up to $n \times m$ docking processes are performed.

In a preferred embodiment, said rigid-body docking is effected by Monte-Carlo simulation, preferably using van der Waals radii of the amino acid residues or atoms of said structure(s) and/or ensemble(s). For a definition of van der Waals radii of residues, see further below. Monte-Carlo simulation is a means of sampling relative orientations of two or more molecules as explained above.

For the purpose of defining the space occupied by a given molecule and for determining accessibility, it is necessary to define the size of the constituent atoms or constituent building blocks (such as amino acids). To this end, van der Waals (vdW) radii are preferably used.

As regards accessibility, the definition thereof is preferably on the basis of vdW radii as well. Here it is preferred to define a contact to occur (or equivalently accessibility to exist) when two building blocks, two amino acids or two atoms are at or closer to each other than a given threshold, said threshold preferably being two times the sum of their respective vdW radii. Such contact (or accessibility) threshold definition may obviously be fine-tuned. For example, threshold values may be between the sum of the vdW radii and 5 times said sum such as between 1.5 times and 3 times said sum.

The term "building block" embraces amino acids, PTMs and atoms as well. In other words, for each constituent group ("building block") of an antigen, antibody or fragment thereof, accessibility may be determined at the level of atoms or that of larger moieties such as amino acids and PTMs. For example, if a PTM is a glycan, it may be represented at the atomic level of detail or at the monosaccharide level with a corresponding effective van der Waals radius for each monosaccharide. If an amino acid is modified with a smaller moiety, the modified amino acid as a whole may be represented by a single entity. For means and methods of representing residues as a whole as opposed to at atomic level, see below.

The term "contact" refers to intermolecular contacts, i.e., between antigen and antibody (or fragment thereof). Contacts are binary variables. For each building block-building block pair, and for each pairing of an antigen structure with an antibody or fragment structure, a contact is zero if the building block-building block distance is greater than the threshold value, and one if said distance is equal or smaller than said threshold.

In a particularly preferred embodiment, accessibility is quantified by counting contacts between antigen and antibody or fragment thereof as they occur during said Monte-Carlo simulation.

Preferably, said contacts are (i) atom-atom contacts; and/or (ii) residue-residue contacts, preferably amino acid residue-amino acid residue contacts. As stated above, in either case, vdW radii are preferably used for defining a contact. vdW radii of atoms are well-established and values are available from the literature; see, for example, Batsanov Inorganic Materials 37: 871-885 (2001). As regards vdW radii of amino acid residues, a preferred definition can be found in Kim and Hummer, J Mol Biol 375: 1416-1433 (2008). This provides for a coarse-grained approach which allows better computational efficiency. The effective vdW radius of a moiety comprising more than one atom can be calculated from the vdW volumes of the constituent atoms while assuming sphericity of the residue. Further information in this respect can also be found in Creighton TE. Proteins: Structures and Molecular Properties. 2nd Ed. W.H. Freeman and Company; New York: 1993.

Preferably, contacts involving post-translational modifications are (i) not counted; or (ii) counted only for a pre-defined subset of said post-translational modifications. A preferred pre-defined subset of PTMs are those which are present with a high degree of consistency in the presented antigen and/or whose chemical identity shows little or no variation. "Consistency" in that context may refer to conservation across species, or conservation across tissues (noting that also within a species PTMs may vary according to tissue). There may also be structural criteria for consistency, for example a well-defined density in a crystal structure or a cryo-electron microscopic image.

In other words, in one preferred embodiment, only contacts between amino acids or their constituent atoms are considered, while amino-acid/PTM and PTM/PTM contacts are not.

In a further preferred embodiment, accessibility in terms of step (c) is accessibility of the amino acid residues of said antigen to the amino acids of the complementarity determining regions (CDRs) of said antibody/ies or fragment(s) thereof.

This provides for focusing on those regions of the antibody or fragment where contacts are considered to be of particular relevance in the context of predicting site-specific mutation activity or immune evasion.

In a further preferred embodiment, said amino acid residues of said antigen are, or are part of, known or predicted epitopes.

This allows for factoring in knowledge, to the extent available, about epitopes, preferably cognate epitopes for the antibody or fragment under consideration.

In a further preferred embodiment, said antigen is an antigen of a pathogen, preferably a mammalian, more preferably human pathogen or an antigen otherwise associated with a disease such as a cancer-associated antigen.

Especially pathogens are known or expect to evolve and mutate rapidly, especially under selection pressure, said pressure for example being exerted by components of the immune system such as antibodies.

In a further preferred embodiment, said pathogen is a virus or a bacterium and/or said antigen is an antigen presented on the surface of said pathogen.

In a further preferred embodiment, said virus is a coronavirus such as SARS-CoV-2, preferably a variant of concern (VoC) of SARS-CoV-2 such as omicron variants, including BA.1, BA.2, BA.4, and BA.5.

While the invention is not limited in that respect, it is of note that outstanding performance has been achieved for SARS-CoV-2 which moved into the focus of attention after the outbreak of the worldwide Covid-19 pandemic.

Related thereto, it is particularly preferred that said antigen is the spike protein of SARS-CoV-2. The inventors could demonstrate that mutations in the spike protein of SARS-CoV-2 variants, including Omicron variants, concentrate in regions where access to antibodies is not hindered by glycans. More specifically, the method of the present invention was capable of identifying candidate mutations that, if added to Omicron BA.1, would further increase immune evasion.

Of these, a region around residue 252 in the N-terminal domain (NTD) stands out, which has already been deleted in the Lambda variant.

In a further preferred embodiment, said mutation activity is associated with immune evasion, in particular in response to antibody/ies or fragments thereof such as defined above. In the Examples, use has been made of the three-dimensional structure of the Fab fragment of antibody CR3022 (pdb accession No. 6W41).

In a second aspect, the present invention provides a use of a computer-generated ensemble of three-dimensional structures of an antigen to predict site-specific mutation activity of said antigen.

Preferred embodiments of the method of the first aspect define, where applicable, preferred embodiments of the use of the second aspect.

It is preferred that furthermore use is made of at least one *in silico* structure of at least one antibody or fragment thereof, preferably of an ensemble thereof.

A preferred means of generating ensembles is MD simulation.

In a third aspect, the present invention provides a data processing device comprising means for carrying out the method of the first aspect or the use of the second aspect.

Such data processing device may be implemented as a computer, a personal computer, a tablet, a workstation or a smartphone.

In a fourth aspect, the present invention provides computer program comprising instructions, which, when the program is executed by a device of the third aspect, cause said device to carry out the method of the first aspect or the use of the second aspect.

In a fifth aspect, the present invention provides a computer-readable medium comprising instructions which, when executed by a device of the third aspect, cause said device to carry out the method of the first aspect or the use of the second aspect.

The Figures show:

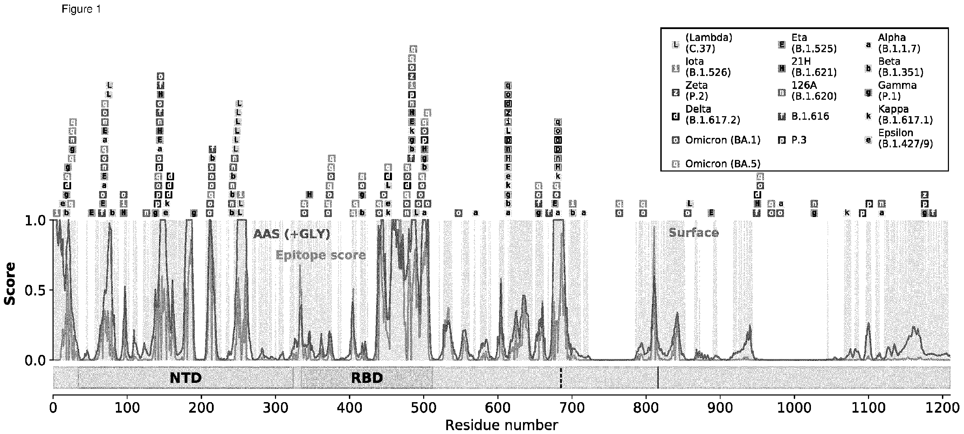

**Figure 1:** SARS-CoV-2 spike mutations and accessibility scores. Mutated residues in spike variants are marked by grey squares and symbols above the plot. The consensus epitope score of Sikora et al., loc. cit. is shown as medium-light gray line with medium-light shading underneath, and the AAS considering glycans as dark gray line with dark shading underneath. Light grey shading in the plot extending to score=1 indicates surface residues as listed in Table A in S1 Text. Variants are distinguished by shade and symbol, as indicated in the legend. The NTD and the RBD are indicated at the bottom, as are the S1/S2 furin cleavage site (vertical dashed line) and the S2' site (solid vertical line).

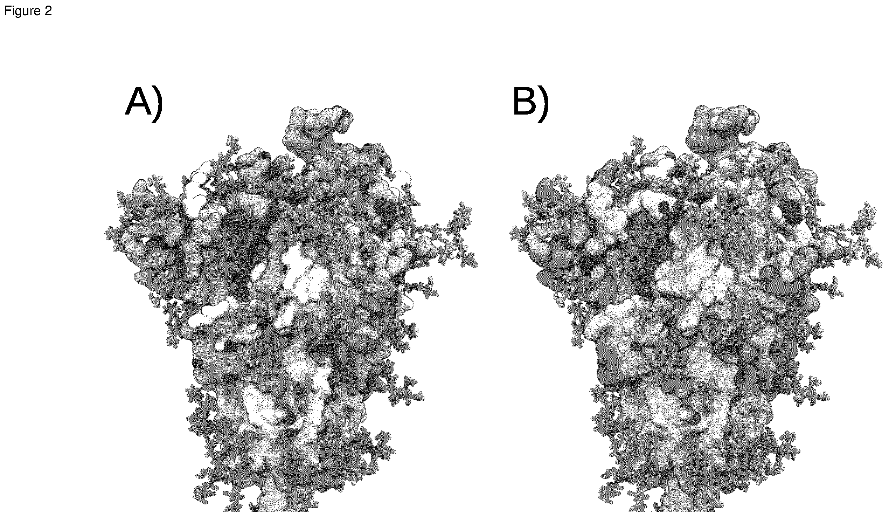

**Figure 2:** Mutations in the spike protein of SARS-CoV-2 variants concentrate in regions of high epitope and accessibility scores. Dark spheres show positions of Omicron BA.1 and BA.5 mutations, and bright spheres those in earlier variants. Intensity of surface shading in (A) and (B) indicates high consensus epitope score and high AAS, respectively.

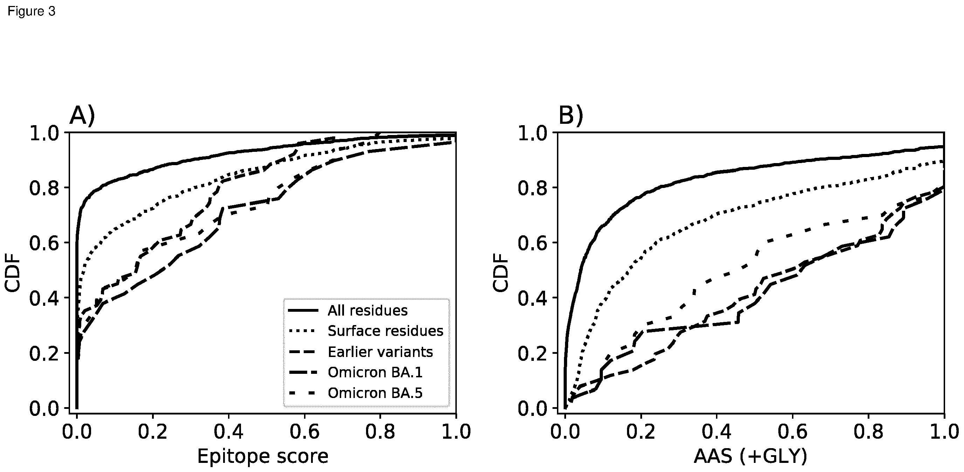

**Figure 3:** Epitope and antibody accessibility scores discriminate between spike mutation sites and generic surface residues. CDFs of (A) the epitope consensus score and (B) the AAS considering glycans and calculated for all spike residues (continuous), surface residues (dotted), sites of mutations in Omicron BA.1 (dash-dotted), Omicron BA.5 (sparse dashes), and earlier variants (dashed). Kolmogorov-Smirnov (KS) statistic described in the text corresponds to the maximum vertical gap between the CDFs for Omicron BA.1 mutation sites and surface residues, respectively. The corresponding P-values for the epitope score (A) and the AAS (B) are 0.016 and $1.5 \times 10^{-5}$, respectively.

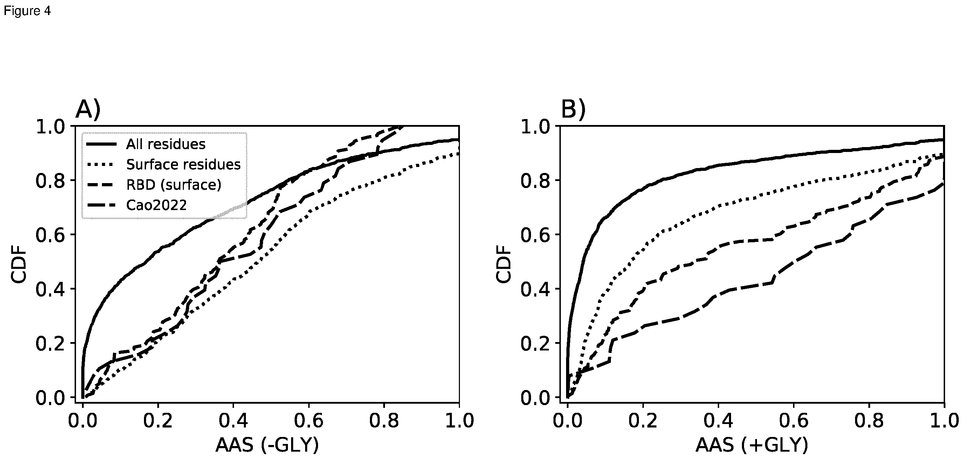

**Figure 4:** Immune escape mutations have high accessibility scores. CDFs of AAS are shown for mutations that were shown experimentally by (Cao et al., Nature 602(7898): 657-663) to reduce binding to neutralizing antibodies (dash-dotted). For reference, we show AAS distributions of surface residues (dotted) and all residues (continuous) of spike, as well as all RBD surface residues (dashed). Score distributions were calculated (A) without glycans and (B) with glycans. The KS statistic is the maximum vertical gap between the respective CDFs and indicated by arrows.

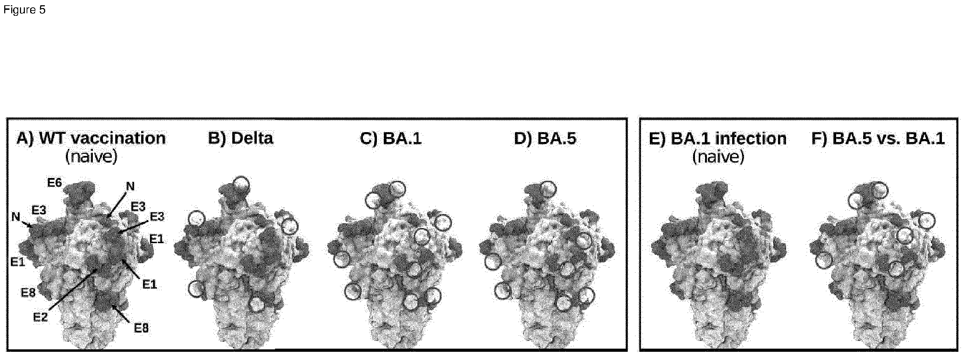

**Figure 5:** Mutations in emerging variants lower antibody access score on surface of SARS-CoV-2 spike relative to wild-type (WT) and previous variants. Spike renders with brightness indicating AAS (white: low AAS to grey: high AAS). (A, E): "Naive" antibody access by antibodies produced after (A) first WT vaccination (no previous infection) or (B) first infection by any variant (no previous vaccination). We assume here that all variants confer approximately the same "native" accessibility to antibodies, and renders in (A) and (E) are identical. (B-D) Calculated reduction of AAS for antibodies produced against WT vaccination. (F) Calculated reduction of AAS for antibodies produced against BA.1 infection. Circles indicate notable reductions in AAS. Regions are labeled according to epitope candidates in Sikora et al., loc. cit.. N: N-terminus. In these renders, the AAS was set to zero for amino acids within 1 nm of any amino acid that was mutated with respect to the reference sequence.

The Examples illustrate the invention.

**Example 1**

Materials and Methods

*Antibody accessibility score*

Extensive rigid-body docking of the Fab fragment of antibody CR3022 (PDB ID: 6W41) to probe the steric accessibility of the spike surface for antibody binding have been performed.
In short, rigid-body docking of a coarse-grained model was performed using the simulation procedures described herein above. In the Monte Carlo docking simulations, we counted the contacts between residues of spike and the complementarity-determining region of the Fab. Structures that clashed with glycans were excluded in the calculation of scores "with glycans" (+gly). The counts were smoothed with a three-dimensional Gaussian filter ($\sigma$ = 0.5 nm) based on the simulation starting structure. The resulting contact counts were then mapped linearly on the interval [0, 1], yielding the AAS. The 5% and 95% percentiles were used for the lower and upper limit of the linear mapping.

*Rays accessibility score*

The rays accessibility scores were based on quasi-isotropic illumination of the spike protein from light rays emanating from the surrounding of the protein, as described in detail in [14]. Glycans were considered (+gly) or not considered (-gly) during illumination. The raw ray hits were used to define general surface residues (see below). For all other results described here, the rays scores were smoothed with a three-dimensional Gaussian filter ($\sigma$ = 0.5 nm) and linear mapping on the interval [0, 1], as for the AAS.

*Spike surface residues*

We defined the surface residues of spike based on the rays accessibility data of unglycosylated spike from Sikora et al., *loc.cit.* We used a reference spike structure in which one RBD was open and two were closed in order to have both open and closed RBDs represented in a single ensemble. A cutoff of >1000 total ray hits across the 2.5 $\mu$s simulation was used for surface residues. For reference, the maximum number of ray hits was about 5000. By imposing a minimum threshold of 1000 hits, we excluded crevices at the surface of spike from the surface definition. The definition of surface residues used here is thus conservative. We used the maximum value of ray hits across the three chains to define the surface accessibility of a particular residue, except in an analysis in which we distinguished between the chains B and C, in which the RBD was closed, and chain A, in which the RBD was open. We extended our analysis up to residue 1206 and excluded the transmembrane domain.

*Variant escapability function*

$$F(v) = \max_{c \in \{A,B,C\}} \left[ \sum_{i \in c} \left( S(i) \left[ 1 - \prod_{j \in M(v)} (1 - \theta_{ij}) \right] \right) / \sum_{k \in c} S(k) \right] \quad (1)$$

The escapability F(v) of a SARS-CoV-2 variant v from modulation of dynamically accessible surface residues was defined as the sum of the antibody accessibility scores S(i) of all residues within 1 nm of a mutated residue, where the maximum is over the chains A, B, and C; the sums run over spike surface residues i and k, respectively; the product runs over the set M (v) of all mutated positions j in the three chains; and $\theta_{ij}$ = 1 if the two residues i and j are within a cutoff distance of 1 nm in the simulation starting structure, and $\theta_{ij}$ = 0 otherwise. The distance was measured at the positions of the $C_\alpha$ atoms in the starting structure. The analysis was done separately for the three protein chains.

*Variants and mutation sites*

In our analysis, we included Omicron and the earlier variants Alpha, Beta, Gamma, Delta, Epsilon, Zeta, Eta, Iota, Kappa, Lambda, Mu, B.1.620, B.1.616, and P.3. As the most recent VoC, we analyzed Omicron separately from the earlier variants, including Delta. We used the main strain of Omicron (BA.1) and the recently emerged BA.5 sublineage in our analysis. Mutation sites present in multiple variants entered only once into the combined set of "earlier variants". Deletions entered at the position of each deleted amino acid. Insertions entered with a single count at the position of the insertion. In the analysis, we included only the surface residues because mutations in the spike interior are unlikely to be driven by the evasion of the antibody immune response. We used the same surface definition as in the calculation of the AAS.

*Statistical assessment*

We used the two-sample KS test to assess the significance of the differences in the distribution of epitope and accessibility scores. For this, we determined the KS statistic, $KS = \max S \mid P(s < S) - Q(s < S) \mid$, as the extremal distance of the respective CDFs of the scores, $P(s < S)$ in surface sites and $Q(s < S)$ in the respective set of variant mutation sites. Sample sizes are the number of surface residues and the number of mutation sites in the set, respectively. We report the KS statistic, the sample sizes, and the corresponding P-values in a two-sample KS test.

**Example 2**

Results

*Spike mutations cluster in regions of predicted epitope candidates*

In Sikora et al., *loc. cit.,* MD simulations of membrane-anchored SARS-CoV-2 spike and bioinformatic tools to identify epitope candidates on spike were used. We defined a consensus epitope score that integrated information on the surface accessibility of glycosylated spike, its structural rigidity, sequence variation at the onset of the pandemic, and a sequence-based bioinformatic epitope predictor. Applied to the MD simulation trajectories of spike, we identified nine distinct regions at the surface of spike with high epitope score. The steady emergence of new SARS-CoV-2 variants allowed us to test these computational predictions by comparing the reported sites of mutations in the spike protein of the variants with the predicted epitopes. Here, we assumed that spike surface mutations are to a significant degree driven by antibody immune evasion. Throughout this work, we treated the Omicron variants and earlier variants separately, as defined in the Methods. By comparing the mutation sites in the SARS-CoV-2 variants to the regions with high predicted epitope score, we found that the first eight of the predicted nine epitope candidates are affected by mutations in Omicron BA.1 or at least one of the earlier variants (Fig 1 and Fig 2A,C). Most of the mutations concentrate in the RBD and in the NTD; however, there is significant mutational activity also outside these regions. For a quantitative assessment of our epitope predictions in light of variant mutational activity, we compared the consensus epitope scores of mutated residues on the surface of spike to those of all surface residues. The cumulative distribution functions (CDFs) of the consensus epitope score reveal a significant shift towards higher scores for residues mutated in spike variants compared to all spike residues (Fig 3A); i.e., mutations occurred with high probability in regions identified as likely epitopes for antibody binding. The score distribution of mutation sites is also shifted significantly to larger values compared to surface residues on spike (for definition of surface residues, see Example 1). The P-values for the KS statistic of Omicron BA.1 and earlier variants with respect to surface residues are 0.016 and 0.010, respectively, and thus significant. A high epitope consensus score (Sikora et al., *loc. cit.*) is correlated with the occurrence of mutations in the spike protein of SARS-CoV-2 variants.

*Antibody accessibility score as predictor of emergent mutations in variants of concern*

The inventors aimed to construct a simpler and more powerful predictor of mutation activity in variant spike based on surface accessibility alone, i.e., without including structural rigidity, sequence conservation, and sequence signatures. We compared three surface accessibility scores, "rays", "antibody accessibility score" (AAS, termed "docking" in Sikora et al., *loc. cit.*), and the solvent-accessible surface area (SASA), with and without including surface glycosylation. The rays score quantifies the accessibility in terms of the surface illumination by diffuse light; see Example 1 above and Sikora et al., *loc. cit.* The AAS measures how accessible each residue is to an antibody-fragment probe. SASA defines the solvent-accessible surface with a spherical probe (radius: 0.14 nm).

All surface accessibility scores were determined for fully glycosylated spike structures collected during $4 \times 2.5$ $\mu$s MD simulations. In this way, they account for the dynamic shielding by glycans. In the following, we evaluate the different scores for variant mutation sites at the surface of spike and compare them to the scores of generic surface residues. Here, we assume that mutations in the interior of spike are driven by factors other than antibody binding. The rays score without including surface glycosylation was used for the definition of general surface residues of spike. Compared to conventional surface definitions based on SASA, our rays analysis does not consider deep crevices as surface-exposed. Thus, the estimated difference in score distributions of mutated residues versus surface residues is quite conservative in the sense that with a more generous definition, even larger differences in the score distribution could be achieved. Consistently across variants, the AAS surprisingly emerged as the top-performing score (Fig 3B, Table 1 below).

**Table 1.** Statistics of rays, antibody accessibility, and SASA scores in discriminating variant mutation sites from surface residues

|  | Rays (-gly) | Rays (+gly) | AAS (-gly) | AAS (+gly) | SASA (-gly) | SASA (+gly) |
|---|---|---|---|---|---|---|
| KS(EV-S) | 0.38 | 0.39 | 0.37 | 0.43 | 0.20 | 0.22 |
| P(EV-S) | $9.0 \times 10^{-7}$ | $3.8 \times 10^{-7}$ | $2.1 \times 10^{-6}$ | $2.3 \times 10^{-8}$ | 0.036 | 0.019 |
| KS(BA.1-S) | 0.36 | 0.43 | 0.28 | 0.45 | 0.18 | 0.19 |
| P(BA.1-S) | $8.0 \times 10^{-4}$ | $3.1 \times 10^{-5}$ | 0.021 | $1.5 \times 10^{-5}$ | 0.31 | 0.25 |
| KS(BA.5-S) | 0.25 | 0.30 | 0.21 | 0.33 | 0.21 | 0.24 |
| P(BA.5-S) | 0.041 | $9.6 \times 10^{-3}$ | 0.14 | $2.3 \times 10^{-3}$ | 0.14 | 0.059 |
| KS(BA.5-BA.1-S) | 0.35 | 0.52 | 0.27 | 0.58 | 0.22 | 0.30 |
| P(BA.5-BA.1-S) | 0.015 | $4.2 \times 10^{-5}$ | 0.10 | $1.8 \times 10^{-6}$ | 0.2 | 0.064 |

Listed in the Table above are the KS statistic and the corresponding P-value obtained for the distributions of the rays score, antibody accessibility score (AAS), and SASA score when comparing generic surface residues (S; Table A in S1 Text) to surface mutation sites in Omicron BA.1 (Table B in S1 Text), Omicron BA.5, and earlier variants (EV). Scores were calculated with glycans (+gly) and without glycans (-gly). Sample sizes N were 29 mutations for Omicron BA.1, 30 mutations for Omicron BA.5, 51 mutations for earlier variants, 19 mutations differing between BA.1 and BA.5, and 620 generic surface residues.

We used the KS statistic to quantify the power of the different scores to distinguish between sites of mutations on the surface of spike and general surface residues of spike. The P-values of the KS test for AAS with glycans with respect to all surface residues are substantially better than for the earlier epitope score. For Omicron BA.1 analyzed with AAS and glycans, we obtained a P-value of $1.5 \times 10^{-5}$, and for the earlier variants a P-value of $2.3 \times 10^{-8}$. By contrast, the simpler rays score was somewhat less discriminatory (Table 1). The commonly used SASA score was least suited to distinguish between mutated and general surface residues, with an insignificant P-value of 0.31 for Omicron BA.1.

Omicron BA.5 shows slightly lower AAS value distributions (Fig 3B) compared to Omicron BA.1 and earlier variants. Remarkably however, the set of residues differing between the BA.1 and BA.5 variant shows a very strong shift towards high antibody accessibility for all metrics that include glycan shielding (BA.5-BA.1-S in Table 1).

*The absence of glycan coverage is a determinant of mutation activity*

Next, we wondered if glycosylation indeed plays a central role in steering the antibody-based immune defence, as we had assumed in the construction of the original epitope score of Sikora et al., *loc. cit.* The CDFs of the AAS and the rays score have been calculated without including glycosylation. In Table 1, we compare the KS statistic and the corresponding P-values as a measure of the discriminatory power of the AAS and rays score between sites of mutations at the surface of spike in major variants, and generic surface sites. We find that accounting for glycosylation substantially improves the discriminatory power of the AAS, and somewhat improves the discriminatory power of the rays score. For the mutation sites in Omicron BA.1 and earlier variants assessed with the AAS against all surface sites, including glycosylation improved the P-value from 0.02 to $1.5 \times 10^{-5}$ and from $2.1 \times 10^{-6}$ to $2.3 \times 10^{-8}$. With the rays score and glycosylation included, the P-value of mutation sites in Omicron BA.1 improved from $8.0 \times 10^{-4}$ to $3.1 \times 10^{-5}$, and in earlier variants from $9.0 \times 10^{-7}$ to $3.8 \times 10^{-7}$. Accounting for the dynamic glycan shield thus substantially improves the discriminatory power of the AAS, and to a lesser degree of the rays score.

*Impact of protein and glycan dynamics*

Next, we wondered whether the sampling of protein and glycan dynamics is essential to discriminate variant mutation sites from generic surface residues. To this aim, we calculated the rays score for the static starting structure of the MD simulation with and without glycosylation. Here, we added glycans to the protein structure in a post-processing step with the software GlycoSHIELD (doi.org/10.1101/2021.08.04.455134). GlycoSHIELD is used to attach glycan structures to proteins at predefined sequons by drawing glycan conformers from a simulation library. Interestingly, we found that a single unglycosylated spike protein structure analyzed with the rays score suffices to distinguish between mutated residues and surface residues for Omicron BA.1 and earlier variants, but not for Omicron BA.5, with P-values of 0.021 for Omicron BA.1, 0.47 for Omicron BA.5, and $8.3 \times 10^{-5}$ for earlier variants. The score becomes significant for Omicron BA.5 and is improved further for Omicron BA.1 and earlier variants by sampling glycan motions. We used GlycoSHIELD (cutoff 3.5 Å, coarse-grained mode, glycan types as defined in doi.org/10.1101/2021.08.04.455134) to construct an ensemble of 160 spike chains with different glycan conformers grafted onto the static protein structure. With this ensemble we obtained P-values of $1.9 \times 10^{-4}$, 0.039, and $2.9 \times 10^{-6}$, respectively, for Omicron BA1, Omicron BA.5, and earlier variants. As expected, we achieved the best performance by sampling both protein and glycan motions by taking the structures of the MD simulation (Table 1), with P-values of $3.1 \times 10^{-5}$, $9.6 \times 10^{-3}$, and $3.8 \times 10^{-7}$, respectively. We expect this trend to hold for the AAS. Whereas expensive MD simulations are needed to get the best predictive power, mean field approaches such as GlycoSHIELD still ensure accurate predictions at a fraction of the computational cost. This could prove valuable for high-throughput studies across a wide range of sequences and structural models for viral and non-viral targets.

*Immune escape mutations have high accessibility scores*

A recent experimental study (Cao et al., Nature 602(7898): 657-663 (2022)) identified a set of spike mutations that severely weakened the binding of neutralizing antibodies. In Fig 4, we show that these mutation sites have a substantially higher AAS than general surface residues. With glycosylation considered, this shift results in a P-value of $4.6 \times 10^{-5}$. Even if the analysis is restricted to the RBD, there is a noticeable shift of the mutation sites to higher AAS values compared to RBD surface residues (dash-dotted and dashed lines, respectively, in Fig 4). We conclude that the comparably simple surface accessibility score presented here robustly identifies spike mutation sites important for immune escape.

*Omicron differs from earlier variants in cumulative score*

We also wondered whether Omicron differed from earlier variants in terms of the spike surface mutations. The Omicron variants have large phylogenetic distance to other major variants and are not part of the antigenic cluster of previous VoC. However, the distributions of the AAS of Omicron BA.1 and earlier variants in aggregate are similar (Fig 3B). In contrast, the AAS distributions of BA.5 are slightly shifted to lower values for most scores. In a more fine-grained view, we find similar accessibility score distributions for all variants. Thus, the accessibility of individual mutated residues in Omicron is, on average, not markedly different from those in earlier variants. What is different, however, is that the total number of mutations in the two Omicron variants considered here is larger, and thus the expected aggregate effect on immune evasion.

*Escapability of SARS-CoV-2 variants*

To quantify the aggregate effect of the mutations in a variant, we introduced an "escapability" score. The escapability score F aims to quantify the degree to which the mutations in a variant result in loss of binding to antibodies raised to WT spike, as in current vaccines. Motivated by our observation that the mutation sites concentrate in regions with high AAS, F measures the weighted fraction of antibody-accessible surface modified by mutations with respect to the reference sequence used, e.g., in vaccination. We extend the effect of a mutation to a 1-nm radius about the mutated site to account for the fact that mutations will perturb the structure and chemistry around the mutation sites. The escapability score F is thus defined in eq. 1 (Example 1) as the sum of the AAS $S(i)$ over all residues i within 1 nm of a mutation site. For simplicity, we do not attempt to qualify the mutations by the change in amino-acid chemistry, which clearly are important for specific binding. For instance, a broad screen of antibodies revealed subtle dependences of their ability to neutralize the Omicron variants on mutations in the mapped epitopes. By definition, the score of WT spike is zero, and the highest possible escapability score is 1.0. With this definition, we found that Omicron BA.1 and BA.5 have substantially higher escapability scores F compared to the other past and present VoC Alpha, Beta, Gamma, and Delta (Table 2).

**Table 2.** Escapability score F of SARS-CoV-2 variants.

| chain | Alpha | Beta | Gamma | Delta | BA.1 | BA.5 | BA.5 vs. BA.1 |
|---|---|---|---|---|---|---|---|
| overall | 0.18 | 0.26 | 0.25 | 0.23 | 0.52 | 0.48 | 0.40 |
| A | 0.18 | 0.22 | 0.22 | 0.19 | 0.52 | 0.48 | 0.40 |
| B | 0.18 | 0.26 | 0.25 | 0.23 | 0.41 | 0.46 | 0.31 |
| C | 0.17 | 0.18 | 0.16 | 0.17 | 0.43 | 0.39 | 0.33 |

The escapability scores calculated with AAS and glycans are listed for individual chains and overall.

Moreover, Omicron variants differ from the earlier variants by having a substantially higher escapability score for chain A, which has the RBD in an open state in our model. Note that RBD-opening of chain A breaks the symmetry between chains B and C, as reflected in slightly different escapability scores for the individual chains in Table 2. The increased escapability of Omicron BA.5 against WT antibodies is remarkable, given that the individual distribution is slightly shifted to lower scores (Fig 3B). The large value of F = 0.52 calculated with AAS and glycans for chain A of Omicron BA.1 compared to F $\approx$ 0.42 for chains B and C is due to the comparably large number of mutations localized in regions of the RBD that become exposed upon opening of the RBD for binding of the ACE2 receptor. By contrast, the escapability scores across the three chains are quite even in the Alpha, Beta, Gamma and Delta variants. Given that the large number of mutations in Omicron spike barely improved its binding affinity to ACE2, our premise that antibody-based immune defense drives selection of spike mutations appears to hold also for mutations in the functionally important RBD. We investigated if the recent Omicron variant BA.5 confers a predicted immune escape advantage over BA.1 following WT vaccination. Table 2 shows that BA.5 does not increase escapability from WT vaccination. We then tested if antibody escape from BA.1 infection in addition to WT vaccination could be a driving factor for the emerging variants BA.2 and BA.5. We therefore assessed the escapability of BA.5 with respect to the BA.1 variants and found a substantial escapability of BA.5 with respect to BA.1 as baseline. Our data therefore indicate that - in addition to factors like infectiousness and incubation time - chemical difference in accessible residues with respect to the previously dominant variant supports antibody escape. To illustrate this point, we rendered the spike protein in gray scale according to the AAS (Fig 5). In Fig 5, the AAS was set to zero for all residues within a distance of 1 nm of any amino acid that differs from the reference sequence. Panels A-D in Fig 5 show the reduction in AAS by variant effects for antibodies produced against WT vaccination (Fig 5A), and panels E-F against BA.1 infection (Fig 5E). (Note that we assume native coverage for all infections or vaccinations to "naïve" patients without previous exposure to SARS-CoV-2 spike. Panels Fig 5A and Fig 5E are therefore identical.) Spike variants BA.1 and BA.5 show stronger suppression of the AAS with WT as reference than Delta (Fig 5B-D). Further, BA.5 causes substantial suppression of the AAS with BA.1 as reference (Fig 5F).

**Claims**

1. A computer-implemented method of predicting site-specific mutation activity of an antigen, said method comprising

    (a) providing *in silico* at least one three-dimensional structure of said antigen, preferably an ensemble of three-dimensional structures of said antigen;
    (b) providing *in silico* at least one three-dimensional structure of at least one antibody or fragment thereof capable of binding to or specific for said antigen, preferably an ensemble of three-dimensional structures of said antibody or fragment thereof;
    (c) determining *in silico* the accessibility of the structure(s) of (a) to the structure(s) of (b), wherein higher accessibility is indicative of higher mutation activity, in particular under selective pressure exerted by said antibody or fragment thereof;

    thereby predicting said site-specific mutation activity.

2. The method of claim 1, wherein said antigen, said antibody and/or said fragment thereof comprise post-translational modifications, preferably selected from N-, O- and S-glycosylation, C-mannosylation, glycation, acylation, alkylation, butyrylation, malonylation, propionylation, sulfation, phosphorylation, ubiquitination, and sumoylation.

3. The method of claim 1 or 2, wherein said ensemble(s) is/are representative of the structural diversity of said antigen, said antibody and/or said fragment thereof at temperatures occurring in a mammalian, preferably human organism and/or at temperatures from 273 to 323 K such as from 300 to 310 K.

4. The method of any one of claims 1 to 3, wherein said structure(s) or said ensemble(s) of (a) and/or (b) are obtained from a database of three-dimensional structures, by crystallography, by NMR spectroscopy, by electron microscopy, by mass spectrometry, by atomic force microscopy, and/or by *in silico* structure prediction.

5. The method of claim 4, wherein conformational sampling of said structure(s) is performed and/or of said ensemble(s) is extended by computer simulation such as molecular dynamics simulation and/or Monte-Carlo simulation, preferably on or representative of a microsecond timescale.

6. The method of any one of the preceding claims, wherein said determining in step (c) is effected by *in silico* docking of said structure or members of said ensemble of (a) with said structure or members of said ensemble of (b).

7. The method of claim 6, wherein said docking is rigid-body docking.

8. The method of claim 7, wherein said rigid-body docking is effected by Monte-Carlo simulation, preferably using van der Waals radii of the amino acid residues or atoms of said structure(s) and/or ensemble(s).

9. The method of claim 8, wherein accessibility is quantified by counting contacts between antigen and antibody or fragment thereof as they occur during said Monte-Carlo simulation.

10. The method of claim 9, wherein said contacts are

    (i) atom-atom contacts; and/or
    (ii) residue-residue contacts, preferably amino acid residue-amino acid residue contacts.

11. The method of claim 9 or 10, wherein contacts involving post-translational modifications are

    (i) not counted; or
    (ii) counted only for a pre-defined subset of said post-translational modifications.

12. The method of any one of the preceding claims, wherein accessibility in terms of step (c) is accessibility of the amino acid residues of said antigen to the amino acids of the complementarity determining regions (CDRs) of said antibody/ies or fragment(s) thereof.

13. The method of any one of the preceding claims, wherein said antigen is an antigen of a pathogen, preferably a mammalian, more preferably human pathogen or an antigen otherwise associated with a disease such as a cancer-associated antigen.

14. Use of a computer-generated ensemble of three-dimensional structures of an antigen to predict site-specific mutation activity of said antigen.

15. A computer program comprising instructions, which, when the program is executed by a data processing device, cause said device to carry out the method of any one of claims 1 to 13.

Figure 1

Figure 2

A)

B)

Figure 3

EP 4 362 026 A1

Figure 4

EP 4 362 026 A1

Figure 5

A) WT vaccination (naive)   B) Delta   C) BA.1   D) BA.5   E) BA.1 infection (naive)   F) BA.5 vs. BA.1

Europäisches
Patentamt

European
Patent Office

Office européen
des brevets

# EUROPEAN SEARCH REPORT

Application Number

EP 22 20 3671

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | HARVEY WILLIAM T ET AL: "SARS-CoV-2 variants, spike mutations and immune escape", NATURE REVIEWS MICROBIOLOGY, NATURE PUBLISHING GROUP, GB, vol. 19, no. 7, 1 June 2021 (2021-06-01), pages 409-424, XP037482862, ISSN: 1740-1526, DOI: 10.1038/S41579-021-00573-0 [retrieved on 2021-06-01] * the whole document * * in particular * * page 412 – page 414; figures 1,2 * ----- | 1-15 | INV. G16B15/30 G16B20/30 G16B20/20 |
| X,D | SIKORA MATEUSZ ET AL: "Computational epitope map of SARS-CoV-2 spike protein", PLOS COMPUTATIONAL BIOLOGY, vol. 17, no. 4, 1 April 2021 (2021-04-01), page e1008790, XP93030327, DOI: 10.1371/journal.pcbi.1008790 * the whole document * * in particular * * sections "results", "methods"; figure 1 * ----- | 1-15 | |
| A | Newby Maddy L. ET AL: "Natural variations within the glycan shield of SARS-CoV-2 impact viral spike dynamics", bioRxiv, 17 August 2022 (2022-08-17), XP093030718, DOI: 10.1101/2022.08.17.504157 Retrieved from the Internet: URL:https://www.biorxiv.org/content/10.1101/2022.08.17.504157v1.full.pdf [retrieved on 2023-03-10] * the whole document * * in particular * * "results and discussion" * ----- -/-- | 1-15 | **TECHNICAL FIELDS SEARCHED (IPC)** G16B |

The present search report has been drawn up for all claims

| Place of search | Date of completion of the search | Examiner |
|---|---|---|
| Munich | 10 March 2023 | Rákossy, Z |

EPO FORM 1503 03.82 (P04C01)

Europäisches Patentamt

European Patent Office

Office européen des brevets

# EUROPEAN SEARCH REPORT

Application Number

EP 22 20 3671

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | Harbison Aoife M. ET AL: "Fine-tuning the Spike: Role of the nature and topology of the glycan shield in the structure and dynamics of the SARS-CoV-2 S", bioRxiv, 1 April 2021 (2021-04-01), XP093030740, DOI: 10.1101/2021.04.01.438036 Retrieved from the Internet: URL:https://www.biorxiv.org/content/10.1101/2021.04.01.438036v1.full.pdf [retrieved on 2023-03-10] * the whole document * * in particular * * "results" and "discussion" sections; figure 1 * | 1-15 | |
| T | Von Bülow Sören ET AL: "Antibody accessibility determines location of spike surface mutations in SARS-CoV-2 variants", , 24 January 2023 (2023-01-24), XP093030866, DOI: https://doi.org/10.1371/journal.pcbi.1010822 Retrieved from the Internet: URL:https://journals.plos.org/ploscompbiol/article?id=10.1371/journal.pcbi.1010822 [retrieved on 2023-03-10] * the whole document * | | TECHNICAL FIELDS SEARCHED (IPC) |

The present search report has been drawn up for all claims

| Place of search | Date of completion of the search | Examiner |
|---|---|---|
| Munich | 10 March 2023 | Rákossy, Z |

EPO FORM 1503 03.82 (P04C01)

# EP 4 362 026 A1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **CAO et al.** *Nature,* 2022, vol. 602 (7898), 657-663
- **TAFT et al.** *Cell,* 2022
- **BAI et al.** *Journal of the American Chemical Society,* 2021, vol. 143 (42), 17646-17654
- **CHEN et al.** *Proceedings of the National Academy of Sciences USA,* 2021, vol. 118 (42), e2106480118
- **STARR et al.** *Cell,* 2020, vol. 182 (5), 1295-1310.e20
- **STARR et al.** *Science,* 2021, vol. 371 (6531), 850-854
- **MANNAR et al.** *Science,* 2022, vol. 375 (6582), 766-764
- **SIKORA et al.** *PLoS Comput Biol,* 2021, vol. 17 (4), e1008790
- **TUNYASUVUNAKOOL et al.** *Nature,* 2021, vol. 596, 590-596
- **BERMAN et al.** *Nucleic Acids Research,* 2000, vol. 28, 235-242
- **LIWO ; SCHERAGA.** *Current Opinion in Structural Biology,* 2008, vol. 18, 134-139
- **JORGENSEN ; TIRADO-RIVES.** *J. Phys. Chem.,* 1996, vol. 100 (34), 14508-14513
- Complexes: Efficient and versatile coarse-grained simulations of protein complexes and their dense solutions. **LINKE M ; QUOIKA P ; BRAMAS B ; KOFINGER J ; HUMMER G.** ChemRxiv. Cambridge Open Engage, 2022
- **ABRAHAM et al.** *SoftwareX,* 2015, vol. 1-2, 19-25
- **AMARO.** *Biophysical Journal,* 2018, vol. 114, 2271-2278
- **FAN et al.** *Quant. Biol.,* 2019, vol. 7, 83-89
- **PINZI ; RASTELLI.** *Int. J. Mol. Sci.,* 2019, vol. 20, 4331
- **KRAWCZYK et al.** *Bioinformatics,* 2014, vol. 30, 2288-2294
- **KIM ; HUMMER.** *J. Mol. Biol.,* 2008, vol. 375, 1416-1433
- **BATSANOV.** *Inorganic Materials,* 2001, vol. 37, 871-885
- **KIM ; HUMMER.** *J Mol Biol,* 2008, vol. 375, 1416-1433
- **CREIGHTON TE.** Proteins: Structures and Molecular Properties. W.H. Freeman and Company, 1993
- **CAO et al.** *Nature,* vol. 602 (7898), 657-663